# EUROPEAN PATENT SPECIFICATION

(11) **EP 2 088 425 B1**
(45) Date of publication and mention of the grant of the patent: **16.10.2019**
(21) Application number: 07832691.5
(22) Date of filing: 28.11.2007
(51) Int. Cl.: G01N 33/487, G01N 27/28, G01N 27/327

(54) **BLOOD TEST DEVICE**
BLUTTESTVORRICHTUNG
DISPOSITIF D'ANALYSE DE SANG

(30) Priority: 30.11.2006 JP 2006323398
(43) Date of publication of application: 12.08.2009
(73) Proprietor: PHC Holdings Corporation, Tokyo 105-8433 (JP)
(72) Inventor: SHINOHARA, Noriyuki, Ehime 791-0395 (JP); MORI, Masakazu, Ehime 791-0395 (JP); KUROKAWA, Koya, Ehime 791-0395 (JP)
(74) Representative: Grünecker Patent- und Rechtsanwälte PartG mbB
(86) International application number: PCT/JP2007/072969
(87) International publication number: WO 2008/066080

(56) References cited:
- WO-A1-01/61341
- WO-A1-93/09710
- WO-A2-01/08551
- DE-T2- 69 811 914
- JP-A- 2002 013 268
- JP-A- 2002 311 022
- US-A1- 2005 265 094
- S F Clarke ET AL: "A history of blood glucose meters and their role in self-monitoring of diabetes mellitus", , 1 January 2012 (2012-01-01), XP055295416, Retrieved from the Internet: URL:http://citeseerx.ist.psu.edu/viewdoc/d ownload?doi=10.1.1.468.2196&rep=rep1&type= pdf [retrieved on 2016-08-15]
- Abbott Diabetes Care: "Owner's Booklet, Manual del propietario: FreeStyle Freedom Blood Glucose Monitoring System, Sistema de monitoreo de glucosa en sangre", , 1 November 2005 (2005-11-01), XP055295413, Retrieved from the Internet: URL:https://www.northcoastmed.com/pdf/manu als/freedom_userguide.pdf [retrieved on 2016-08-15]
- Karen Athens ET AL: "Guidelines for the Design of Consumer Products to Increase Their Accessibility to People with Disabilities or Who Are Aging", , 1 January 1992 (1992-01-01), XP055295515, Retrieved from the Internet: URL:http://trace.wisc.edu/docs/consumer_pr oduct_guidelines/consumer.htm#input [retrieved on 2016-08-15]
- Mark M Uslan ET AL: "Accessibility of blood glucose monitoring systems for blind and visually impaired people", Diabetes technology & therapeutics, 1 January 2003 (2003-01-01), pages 439-448, XP055295524, United States DOI: 10.1089/152091503765691947 Retrieved from the Internet: URL:http://online.liebertpub.com/doi/pdf/1 0.1089/152091503765691947
- Matthew Bularzik ET AL: "Accessible Blood Glucose Monitor", , 1 May 2006 (2006-05-01), XP055295530, Retrieved from the Internet: URL:http://www.bme.uconn.edu/bme/sendes/Sp ring06/Team2/final.pdf [retrieved on 2016-08-15]
- OGIZO M.: 'Koten Shikikaku Ijosha ni Taisuru Shikiso-shoku ni yoru Kondo Iro Kiseki no kento' JOURNAL OF JAPANESE OPHTHALMOLOGICAL SOCIETY vol. 97, no. 3, 10 March 1993, pages 411 - 418, XP008110436
- SUZUKI I.: 'Shikikaku Barrier Free tte Nandesuka?' THE JAPANESE JOURNAL OF OPHTHALMIC CARING vol. 6, no. 3, 01 March 2004, pages 228 - 237, 199, XP008110643

## Description

### Technical Field

The present invention relates to a blood test apparatus that can be used easily by people with vision problems such as diabetes patients and old people.

### Background Art

Conventionally, a blood test apparatus is known that has a blood sensor and a blood test apparatus body to which the blood sensor is detachably attached (see, for example, Patent Document 1). FIG.1 shows an example of conventional blood test apparatus 1. Housing 2 of blood test apparatus 1 has a practically rectangular parallelepiped shape. Attaching part 4 is provided in side surface 2a of housing 2 and blood sensor 3 (hereinafter also referred to as "sensor") is inserted into attaching part 4. Attaching part 4 and housing 2 usually have similar colors. Further, display section 5 and operation button 6 are provided in upper surface 2b of housing 2. Display section 5 and operation buttons 6 are connected to an electrical circuit section (not shown).

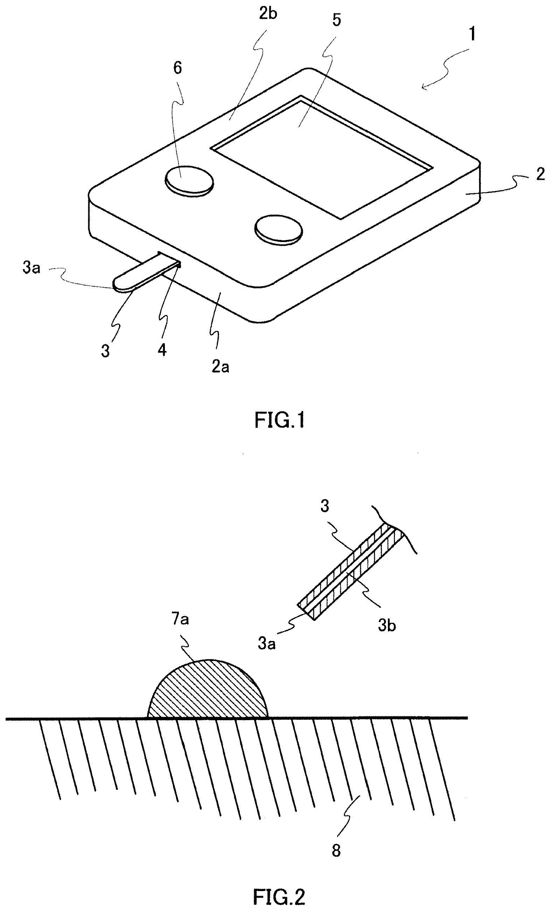

As shown in FIG.2, sampling slot 3a for sampling blood 7 is provided in one end of sensor 3. Sampling slot 3a is coupled to a detecting section through passage 3b. Detecting electrodes are provided in the detecting section. The detecting electrodes are connected to the electrical circuit section through a connector provided in the blood test apparatus body.

The operation of blood test apparatus 1 will be explained. Preparation for blood test is arranged by inserting sensor 3 in attaching part 4 provided in side surface 2a of blood test apparatus 1. After preparation is finished, the patient punctures skin 8 of the patient. As shown in FIG. 2, as a result of this puncturing, blood flows out from skin 8 and forms drop 7a of blood. Sampling slot 3a of sensor 3 is made to contact this drop 7a of blood. Then, blood flows in the detecting section from sampling slot 3a and chemically reacts with a reagent in the detecting section. An electrical signal generated by this chemical reaction is transmitted to the electrical circuit section through a connector. The blood sugar level is calculated in the electrical circuit section. The calculation result is displayed in display section 5. Based on the level displayed in display section 5, the patient injects insulin if necessary.
Another blood test device previously known is called Reflomat.
Patent Document 1: International Publication No.02/44705 Pamphlet

### Disclosure of Invention

### Problems to be Solved by the Invention

Among diabetes patients, there are patients with complications suffering diabetes and diabetic retinopathy together, and old patients with poor sight. In other words, these patients have vision problems. Nevertheless, a conventional blood test apparatus was not easy to operate for these patients with vision problems . That is, if the color of the sensor attaching part of the blood test apparatus and the color of the housing are similar, it is not easy for patients with vision problems to recognize the location of the sensor attaching part and insert the sensor in the sensor attaching part.

Recently, blood test apparatuses are reported in which the color near the attaching part is different from the surface color of the housing (see http:/-/www.abbott.co.jp/medical/product/adc/index.as p?r=1). However, the surface color of these blood test apparatuses is selected taking the design alone into account and is not selected by taking into account simplicity for recognizing the location of the attaching part easier. Some information can be derived from the "Owner's Booklet Manual del propietario: Freestyle Freedom Blood Glucose Monitoring System, Sistema de monitoreo de glucose a en sangre" (2005), retrieved from URL:https://www.northcoastmed.com/pdf/manuals/freedo m_userguide.pdf.

It is therefore an object of the present invention to provide a blood test apparatus that solves such a problem and that can be used easily by diabetic retinopathy patients and old patients with poor sight.

### Means for Solving the Problem

The blood test apparatus of the present invention is defined by the subject matter of claim 1. Preferred embodiments are claimed in the dependent claims.

The inventors have made the present invention by focusing upon that, if the surface colors of parts that need to be operated frequently in a blood test apparatus have higher color values than the color value of the surface color of the housing and have a color that can be identified by eyes with impaired blue cones, even patients with vision problems can operate the parts easily.

### Advantageous Effect of the Invention

According to the blood test apparatus of the present technique, the surface color of the attaching part of the sensor has a higher color value than the surface color of the housing and has a color that can be identified by eyes with impaired blue cones. Preferably, the blood test apparatus of the present invention has operation buttons and, similar to the sensor attaching part, the operation buttons have higher color values than the surface color of the housing and have a color that can be identified by eyes with impaired blue cones. (Hereinafter, the attaching part of the sensor and the operation buttons will also be referred to as "input section" collectively.) Consequently, diabetic retinopathy patients and old people can identify the input section easily and consequently operate the blood test apparatus easily every time. Therefore, the blood sugar level can be controlled easily, so that it is possible to prevent (or delay) the progress of various complications including diabetic retinopathy.

### Brief Description of Drawings

FIG. 1 is a perspective view of a conventional blood test apparatus;
FIG.2 is a cross-sectibnal view explaining use of the blood test apparatus shown in FIG. 1;
FIG. 3 is a perspective view of a blood test apparatus
FIG.4 is a cross-sectional view near an attaching part of the blood test apparatus shown in FIG.3;
FIG. 5 is a perspective view of a blood test apparatus according to an embodiment of the present invention;
FIG. 6 is a perspective view of a blood test apparatus according to another embodiment of the present invention;
FIG. 7 is a perspective view of a blood test apparatus according to another embodiment of the present invention;
FIG. 8 shows an assembly of a sensor that is configured to be attached to the blood test apparatus;
FIG. 9 is a block diagram of an electrical circuit section provided in the blood test apparatus;
FIG.10 illustrates the Munsell color system;
FIG.11 illustrates spectroscopic characteristics of human eyes; and

### Best Mode for Carrying Out the Invention

The blood test apparatus according to the present technique has: (1) a housing; (2) an electrical circuit section that is built in the housing; and (3) an attaching part that is provided in the surface of the housing to attach a blood sensor. Preferably, the blood test apparatus further has (4) operation buttons that are provided in the surface of the housing and that connects with the electrical circuit section.

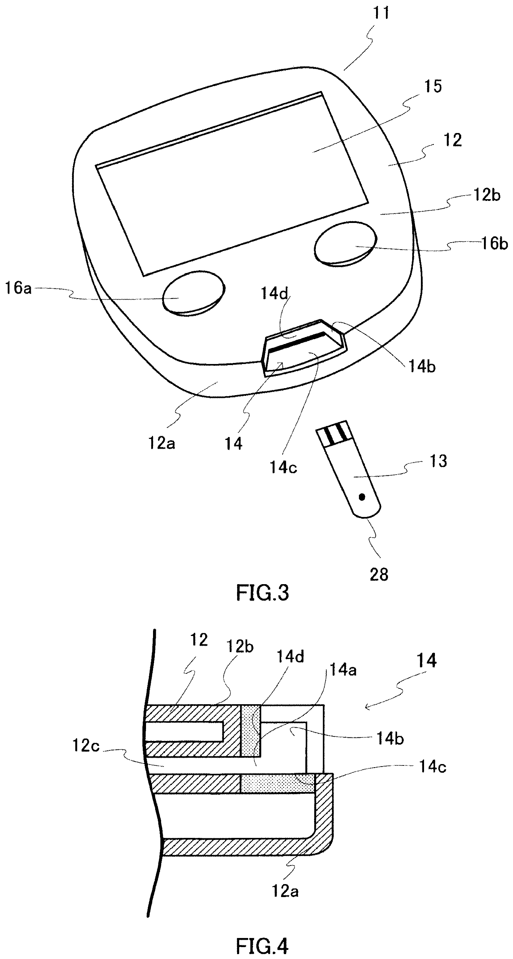

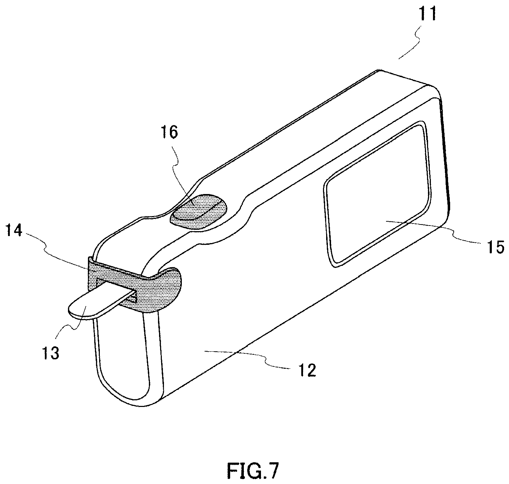

Preferably, the blood sensor attached to the attaching part is detachable from the apparatus body and is replaced every blood test. Further, the blood sensor may be inserted from outside the blood test apparatus in the attaching part every test (see, for example, FIG. 3), and the blood sensor stored in advance in the blood test apparatus may be let out from inside the apparatus to the attaching part every test (see, for example, FIG. 7).

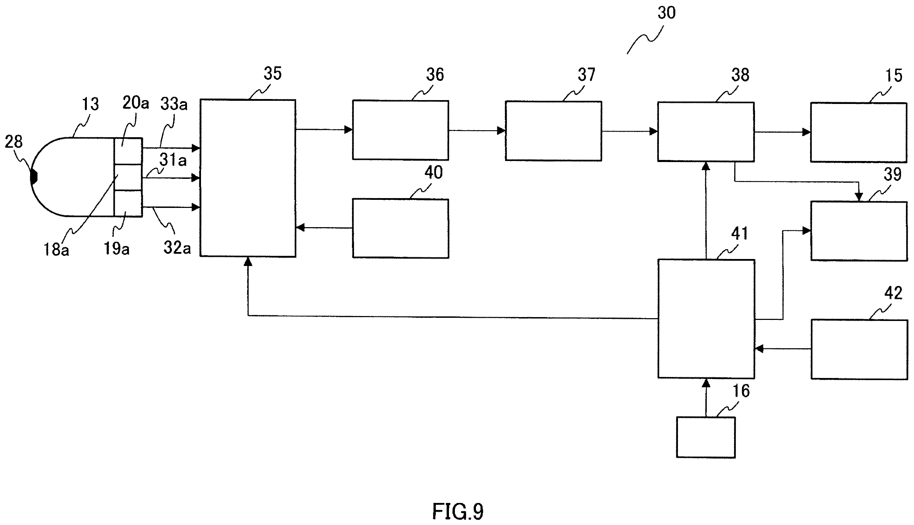

FIG.3 is a perspective view of an example of a blood test apparatus. Blood test apparatus 11 shown in FIG. 3 has: (1) housing 12 of a rectangular parallelepiped shape; (2) attaching part 14 that is arranged in side surface 12a of housing 12; (3) display section 15 and operation button 16 (that refers to operation buttons 16a and 16b collectively) that are arranged in upper surface 12b of housing 12; and (4) electrical circuit section 30 (see FIG.9) that is accommodated in housing 12 and that is connected with display section 15 and operation button 16. Blood sensor 13 is inserted in attaching part 14.

Features of the blood test apparatus of the present invention lie in the surface color of attaching part 14 or operation button 16. That is, the present invention includes features that the surface color of the attaching part or operation button has the color that is readily recognized by diabetic retinopathy patients and the like. The surface of attaching part 14 means at least one of upstanding wall surface 14b, horizontal surface 14c and vertical surface 14d of attaching part 14 in FIG. 3. For example, by maximizing the color value of horizontal surface 14C among upstanding wall surface 14b, horizontal surface 14c and vertical surface 14d, the location of the attaching part is identified more easily.

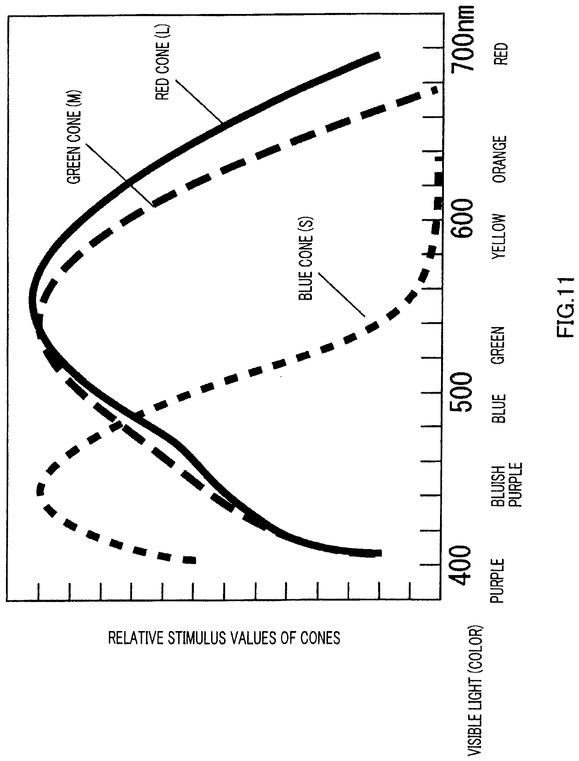

The human retina has two kinds of photoreceptor cells including the rods that function when it is dark and the cones that function when it is bright. As shown in the spectroscopic characteristic diagram of FIG. 11 there are three kinds of cones including the red cones (L), green cones (M) and blue cones (S), and they are mainly sensitive to different wavelengths. These cones react to light of these three kinds of wavelengths and so people recognize colors.

However, because diabetic retinopathy problems damage the cones, the number of cone cells to function decreases. The number of blue cones in particular is originally less than the red cones and the green cones, and accounts for only several percents in the total number of cone cells. Accordingly, the blue cones are susceptible to the influence of the retina problem of the early period and the symptoms of blue color blindness and blue color weakness are likely to develop.

A human with no impaired cones recognize colors in the range from bluish purple, blue, bluish green, green to yellowish green as the blue color, and so the range of the wavelength recognized as the blue color is relatively wide. Accordingly, patients with impaired cones have difficulty in recognizing the green color, and recognizes the yellow color as a similar, whitish color of lower chroma.

In this way, patients with impaired cones can recognize the limited range of colors. Therefore, if the surface colors of parts (the parts which need to be operated will also be referred to as "input section" below) that need to be operated frequently in the blood test apparatus, are not appropriately selected, it is difficult to learn the locations of the parts. Accordingly, the operation of the apparatus becomes difficult, thereby making the blood test complicated.

Examples of the parts (i.e. input section) that need to be operated frequently in the blood test apparatus according to the present invention include the attaching part in which the blood sensor is inserted and the operation buttons for inputting an electrical signal to the electrical circuit section. These parts need to be operated every blood test.

The inventors have focused upon that, if the surface color of the input section is made identifiable for eyes with impaired cones and the color value of the surface color of the input section is made higher than the color values of the surface colors of surrounding parts, patients with impaired cones readily recognize the input section easily.

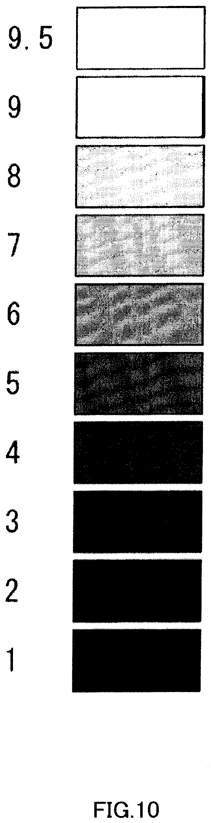

The color value is an indication of the brightness of a color. There is the Munsell color system as a means for converting color values into numerical values. FIG.10 shows the color value table of the Munsell color system. Preferably, the color value of the surface color of the input section of the apparatus according to the present invention defined by the Munsell color system is in the range approximately between 9.5 and 7. On the other hand, preferably, the color values of the surface colors around the input section (for example, the housing) are within a range approximately between 1 and 4.

For example, the surface color of housing 12 of the blood test apparatus shown in FIG.3 is a (dark) color of a low color value, that is, a dark gray color, and the surface colors of attaching part 14 and operation button 16 are (light) colors of high color values, that is, light gray colors. The contrast of colors makes it easier to recognize the locations of attaching part 14 and operation button 16.

A hue refers to the tinge of each color, not including black and white, and red, blue and yellow are the three elements of hues. The hue is represented by the wavelength of the spectrum monochromatic color. The surface color of the input section of the apparatus according to the present invention is in the range from an orange color to a red color, and the wavelengths of an orange color to a red color are in the range from 600 to 700 nanometers. The orange color to the red color are identifiable or readily identified by eyes with impaired cones. Further, preferably, the surface color of the attaching part in which the sensor of the input section is inserted is the orange color (the wavelength of 600 to 675 nanometers) rather than the red color. This is because there are cases where blood adheres to the attaching part of the blood sensor and, if the attaching part is red, adhered blood is hard to see.

By contrast with this, the surface color near the input section (for example, the housing) according to the invention is in the range from a purple color, a blue color to a green color, and, the wavelength of the surface color in the range from the purple color, the blue color to the green color is in the range from 400 to 550 nanometers.

The diabetic retinopathy patients recognize light of the wavelength of 400 to 550 nanometers as a color in the range from the purple color, the blue color to the green color, and recognize this light as a (dark) color of a low color value. Further, these patients recognize light of the wavelength of 600 to 700 nanometers as a color in the range from the orange color to the red color, and recognize this light as a (light) color of a high color value. That is, patients can recognize the difference in the hue and the difference in the color value.

For example, the surface color of housing 12 of the apparatus shown in FIG.3 is in the range from the purple color, the blue color to the green color (i.e. the wavelength of 400 to 550 nanometers) and the surface color of attaching part 14 is the orange color (i.e. the wavelength of 600 to 675 nanometers) . Further, the color value of the surface color of operation button 16 is lower than the color value of the surface color of attaching part 14. Therefore, the surface color of operationbutton 16 is not as distinct as the surface color of attaching part 14 for retinopathy patients. This makes it easier for retinopathy patients to distinguish between attaching part 14 and operation button 16.

As explained above, the orange color has high chroma and can distract attention of retinopathy patients, so that attaching part 14 can be identified easily. Accordingly, diabetic retinopathy patients and old people can identify attaching part 14 easily and use blood test apparatus 11 easily. Consequently, the blood sugar level can be controlled easily, so that it is possible to prevent (or delay) the progress of retinopathy.

Further, blood test using the blood test apparatus is not always performed in a sufficiently bright environment. There may be cases where test is performed outside in a dim environment e.g., the toilet, bathroom, etc. Accordingly, a fluorescent coating material or phosphorescent coating material may be applied to attaching part 14 and operation button 16 of apparatus 11 shown in FIG.3. By so doing, these parts can be identified easily. Further, light emitting diodes of the orange color (the red color is also possible) may be embedded in attaching part 14 and operation button 16. In this case, attaching part 14 and operation button 16 illuminate brightly, so that it is possible to identify them.

FIG.4 is a cross-sectional view near attaching part 14. Attaching part 14 is provided at a corner where side surface 12a and upper surface 12b contact. Attaching part 14 includes horizontal surface 14c formed horizontally with respect to upper surface 12b of housing 12, and upstanding wall surface 14b and vertical surface 14d that are formed vertically with respect to upper surface 12b of housing 12. Attaching part 14 is open at the corner of housing 12 and so can be identified from the side surface 12a side and the upper surface 12b side of housing 12. Consequently, it is possible to identify the location of attaching part 14 easily.

At the location retired into the apparatus from side surface 12a of attaching part 14, there is inlet 14a for allowing sensor 13 to enter inside the apparatus. That is, inlet 14a is a gap between horizontal surface 14c and vertical surface 14d. Inlet 14a is coupled to passage 12c for sensor 13. A connector is provided deep inside passage 12c. Inlet 14a looks black (i.e. dark) due to the shade. Consequently, when the surface color of attaching part 14 is made the orange color, it is possible to identify the location of attaching part 14 easily thanks to the contrast between inlet 14a and attaching part 14.

Preferably, upstanding wall surface 14b (see FIG. 3) of attaching part 14 has a tapered shape from inlet 14a to outside the apparatus. That is, the shape of attaching part 14 becomes narrower toward inlet 14a. Consequently, sensor 13 can be guided to inlet 14a along upstanding wall surface 14b, horizontal surface 14c and vertical surface 14d of attaching part 14 and inserted easily.

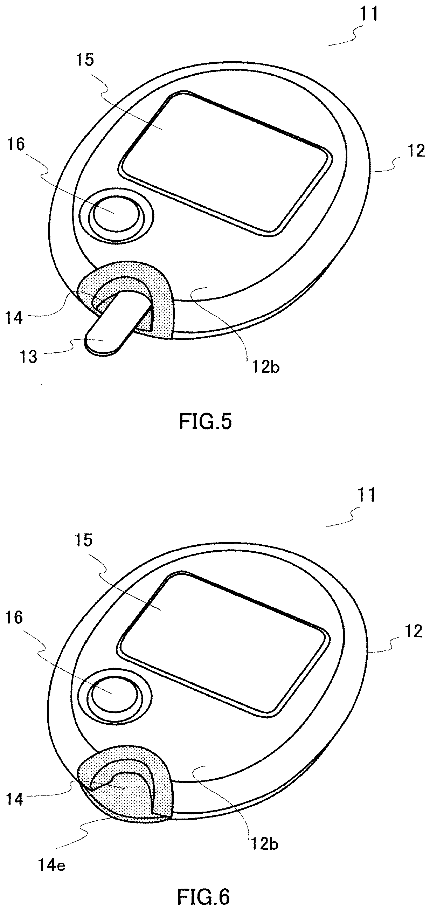

FIG.5 and FIG.6 are perspective views of other examples of blood test apparatus 11 according to the present invention. Attaching part 14 of the blood test apparatus shown in FIG.5 is made bigger than attaching part 14 of the apparatus shown in FIG.3. Further, the hue or color value of upper surface 12b of housing 12 near attaching part 14 of FIG. 5 is changed from the hue or color value of the housing, so that the visibility of attaching part 14 improves. Furthermore, attaching part 14 is shaped to project from upper surface 12b of housing 12, so that attaching part 14 is recognized more easily.

In attaching part 14 of blood test apparatus 11 shown in FIG. 6, the periphery of attaching part 14 projects to form projecting part 14e compared to attaching part 14 of the apparatus shown in FIG.5, and makes a unique shape of attaching part 14. By making a unique shape of attaching part 14 by means of projecting part 14e, the user can recognize the location of attaching part 14 through the tactile sense.

FIG.7 is a perspective view of another example of the blood test apparatus according to the present invention. While the blood test apparatuses shown in FIG. 3, FIG. 5 and FIG. 6 are each used by "inserting" blood sensor 13 from outside the blood test apparatuses to attaching part 14, blood test apparatus 11 shown in FIG.7 "lets out from inside" apparatus blood sensor 13 accommodated in the apparatus to be attached in attaching part 14. Preferably, the visibility of attaching part 14 is high in the apparatus shown in FIG.7, and the hue and color value are selected similar to the apparatus shown in FIG.3. It naturally follows that the hue and color value of operation button 16 shown in FIG.7 may be changed from the hue and color value of housing 12.

In the blood test apparatus, a cartridge formed by stacking a plurality of blood sensors 13 may be stored. Blood sensors 13 are let out from inside the apparatus one by one every test. According to apparatus 11 shown in FIG. 7, the user' s blood sensor 13 needs not be inserted in the attaching part, thereby making the test operation simple . Further, blood test apparatus 11 and blood sensor 13 need not be carried separately.

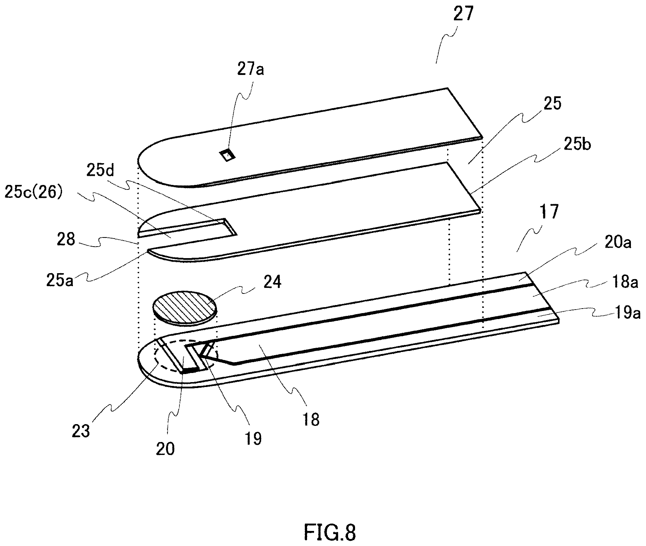

FIG. 8 is an exploded view of the assembly of blood sensor 13. In FIG.8, detecting electrodes 18 to 20 and connection electrodes 18a to 20a derived from detecting electrodes 18 to 20, are arranged on the upper surface of substrate 17. Detecting electrodes 18 to 20 and connection electrodes 18a to 20a are integrally formed by applying laser machining to a conductive layer which is made of gold, platinum or palladium and which is formed in substrate 17 by the sputtering method or vapor deposition method.

Detecting section 23 is formed by detecting electrodes 18 to 20 of substrate 17, and reagent 24 is placed on detecting section 23. The reagent is appropriately selected depending on the kind of blood components to measure. For example, reagent 24 for measuring the blood sugar level can be made by dropping and drying the reagent solution, which is prepared by adding and dissolving PQQ-GDH and potassium ferricyanide .

Spacer 25 made of polyethylene terephthalate is attached on substrate 17 and reagent 24 . Slit 25 is formed in spacer 25 from the front end 25a side toward rear end 25b. Slit 25c exposes detecting section 23. Further, depth part 25d of slit 25c exposes connection electrodes 18a to 20a formed in substrate 17.

When cover 27 (explained later) is overlaid, slit 25c forms passage 26. Front end 25a of spacer 25 serves as sampling slot 28 for blood 7. If the inner wall surface of slit 25c is subjected to hydrophilic treatment, drop 7a of blood (see FIG.2) enters passage 26 from sampling slot 28 by capillary action and is guided to detecting section 23.

Cover 27 is attached on spacer 25. Cover 27 may be made of polyethylene terephthalate. By overlaying substrate 17, spacer 25 and cover 27, passage 26 corresponding to slit 25c is formed. Air hole 27a is formed in cover 27. Air hole 27a and passage 26 communicate through depth part 25d of passage 26.

When blood 7 flows in blood sensor 13 shown in FIG.8 from sampling slot 28, blood 7 is guided at a burst to detecting section 23, in the rate controlled state, by the capillary force of passage 26. Then, blood 7 chemically reacts with reagent 24 placed in detecting section 23.

FIG.9 is a block diagram of electrical circuit section 30 of blood test apparatus 11. In FIG.9, connection electrodes 18a to 20a of blood sensor 13 are connected with switching circuit 35 through connectors 31a to 33a. The output of switching circuit 35 is connected with the input of current/voltage converter 36. The output of current/voltage converter 36 is connected with the input of arithmetic operation section 38 through analogue/digital converter 37 (hereinafter "A/D converter"). The output of arithmetic operation section 38 is connected with display section 15 and transmitting section 39. Display section 15 is a liquid crystal display section, for example. Reference voltage source 40 is connected with switching circuit 35. Reference voltage source 40 may be a ground potential.

Moreover, the output of controlling section 41 is connected with the control terminal of switching circuit 35, arithmetic operation section 38 and transmitting section 39. The input of controlling section 41 is connected with operation button 16 (see 16a and 16b of FIG.3) and timer 42, and may further be connected with a timer (not shown) and memory (not shown) .

The operation of electrical circuit section 30 will be explained. By pressing operation button 16a, power is supplied to electrical circuit section 30. Power may be supplied automatically by detecting inserted sensor 13.

A signal of sensor 13 is inputted to switching circuit 35 through connection electrodes 18a to 20a and connectors 31a to 33a. Controlling section 41 switches switching circuit 35 and connects detection electrode 20 (see FIG.8) with current/voltage converter 36. Further, controlling section 41 connects detecting electrode 18, which serves as a sensing electrode for sensing the inflow of blood 7, with reference voltage source 40. Furthermore, a constant voltage is applied between detecting electrode 18 and detecting electrode 20.

When blood 7 flows in the detecting section in this state, a current flows between detecting electrode 18 and detecting electrode 20. This current is converted into voltage by current/voltage converter 36 and its voltage value is converted into a digital value by A/D converter 37. The digital value is outputted to arithmetic operation section 38 and arithmetic operation section 38 senses that a sufficient amount of blood 7 has flowed in the detecting section.

Next, the amount of blood components (for example, glucose) is measured. First, switching circuit 35 switches according to a command from controlling section 41, and detecting electrode 20, which serves as an active electrode for measuring the amount of blood components, is connected with current/voltage converter 36. Further, detecting electrode 19, which becomes a counter electrode for measuring the amount of blood components, is connected with reference voltage source 40.

When the blood components and the reagent (for example, oxidation reduction enzyme) are made to react, current/voltage converter 36 and reference voltage source 40 may be turned off for a predetermined period (for example, one to ten seconds). Then, after a predetermined period passes, a certain voltage (0.2 to 0.5 V) is applied between detecting electrode 20 and detecting electrode 19 according to a command from controlling section 41. Then, a current flows between detecting electrode 20 and detecting electrode 19. This current is converted into voltage by current/voltage converter 36, and this voltage value is converted into a digital value by A/D converter 37. The digital value is outputted to arithmetic operation section 38. Arithmetic operation section 38 converts the digital value into the amount of glucose components. The amount of glucose components is stored in order in the memory every time measurement is performed. Every time operation button 16b is pressed, the amount of glucose components stored in the memory is displayed in display section 15 in the order the amounts of glucose components are stored.

Further, the amount of blood components may be transmitted from transmitting section 39 to the apparatus that injects insulin. This transmission may be performed using radio waves but may preferably be performed by optical transmission that does not interfere with medical equipment.

The blood test apparatus according to the present invention can be used to measure any blood components and can also be used to measure glucose. Further, by appropriately selecting the reagent in sensor 13, it is possible to measure blood components other than glucose, such as the lactate acid level and cholesterol.

### Industrial Applicability

The blood test apparatus according to the present invention can be used every time easily by diabetic retinopathy patients with vision problems and old people with poor sight, and, consequently, is useful as a blood test apparatus used by patients with vision problems.

## Claims

1. A blood test apparatus (11) comprising:
a housing (12); and
an electrical circuit section (30) built in the housing (12) for analyzing an electrical signal from a blood sensor;
the blood test apparatus (11) having an attaching part (14) for attaching the blood sensor, the attaching part (14) being provided in a surface of the housing (12),
wherein the attaching part (14) includes a horizontal surface (14c), formed horizontally with respect to an upper surface (12b) of the housing (12), and an upstanding wall surface (14b) and a vertical surface (14d) that are formed vertically with respect to the upper surface (12b) of the housing (12),
**characterised in that**
a surface color of a surface of the attaching part (14), where said surface is at least one of the horizontal surface (14c), the upstanding wall surface (14b) and the vertical surface (14d), has a higher color value than a color value of a surface color of the housing (12) around the attaching part (14) and is a color that can be identified by eyes with impaired blue cones,
a hue of the surface color of said surface of the attaching part (14) is in a range from an orange color to a red color with a wavelength of 600 to 700 nanometers, and a color value of the surface color of the attaching part (14) defined by the Munsell color system is between 9.5 and 7,
a hue of the surface color of the housing (12) around the attaching part (14) is in a range from a purple color, a blue color to a green color with a wavelength of 400 to 550 nanometers, and a color value of the surface color of the housing (12) around the attaching part (14) defined by a Munsell color system is between 1 and 4,
the attaching part (14) is shaped to project from the upper surface (12b) of the housing (12),
the horizontal surface (14c) of the attaching part (14) extends to a projecting part (14e) of the attaching part (14) projecting beyond a side of the housing (12), and
the attaching part (14) has a shape that becomes narrower toward an interior of the blood test apparatus (11).

2. The blood test apparatus (11) according to claim 1, further comprising an operation button (16, 16a, 16b) which is connected with the electrical circuit section (30) and which is provided in the surface of the housing,
wherein a surface color of the operation button has a higher color value than the color value of the surface color of the housing around the operation button (16, 16a, 16b) and is the color that can be identified by the eyes with impaired blue cones,
wherein a hue of the surface color of the operation button is in a range from an orange color to a red color with a wavelength of 600 to 700 nanometers, and a color value of the surface color of the operation button defined by the Munsell color system is between 9.5 and 7.

3. The blood test apparatus (11) according to claim 1, wherein a fluorescent material or a phosphorescent agent is applied to the attaching part (14).

4. The blood test apparatus (11) according to claim 2, wherein a fluorescent material or a phosphorescent agent is applied to the operation button (16, 16a, 16b).

5. The blood test apparatus according to claim 1, wherein a light emitting diode is embedded in the attaching part (14).

6. The blood test apparatus according to claim 2, wherein a light emitting diode is embedded in the operation button (16, 16a, 16b).

7. The blood test apparatus (11) according to claim 1 that is adapted to measure a blood sugar level.

8. The blood test apparatus (11) according to claim 1, for using a cartridge with the blood sensors to be stored in the blood test apparatus (11), wherein the attaching part (14) is adapted to pass the blood sensors one-by-one from inside of the blood test apparatus (11).

## Patentansprüche

1. Bluttestvorrichtung (11), mit:
einem Gehäuse (12); und
einem elektrischen Schaltungsabschnitt (30), der in dem Gehäuse (12) eingebaut ist und zum Auswerten eines elektrischen Signals aus einem Blutsensor dient;
wobei die Bluttestvorrichtung (11) ein Befestigungsteil (14) zur Befestigung des Blutsensors aufweist, wobei das Befestigungsteil (14) an einer Oberfläche des Gehäuses (12) vorgesehen ist,
wobei das Befestigungsteil (14) eine horizontale Oberfläche (14c), die in Bezug auf eine obere Oberfläche (12b) des Gehäuses (12) horizontal ausgebildet ist, und eine aufrechtstehende Wandfläche (14b) und eine vertikale Oberfläche (14d) enthält, die vertikal in Bezug auf die obere Oberfläche (12b) des Gehäuses (12) ausgebildet sind,
**dadurch gekennzeichnet, dass**
eine Oberflächenfarbe einer Oberfläche des Befestigungsteils (14), wobei die Oberfläche die horizontale Oberfläche (14c) und/oder die aufrechtstehende Wandfläche (14b) und/oder die vertikale Oberfläche (14d) ist, einen Farbwert hat, der höher ist als ein Farbwert einer Oberflächenfarbe des Gehäuses (12) um das Befestigungsteil (14) herum, und eine Farbe ist, die mit Augen mit beeinträchtigtem blauen Konus erkennbar ist,
wobei ein Farbton der Oberflächenfarbe der Oberfläche des Befestigungsteils (14) im Bereich der Farbe Orangen bis zur Farbe Rot mit einer Wellenlänge von 600 bis 700 Nanometer liegt, und ein Farbwert der Oberflächenfarbe des Befestigungsteils (14), der durch das Munsell-Farbsystem definiert ist, zwischen 9,5 und 7 liegt,
wobei ein Farbton der Oberflächenfarbe des Gehäuses (12) um das Befestigungsteil (14) herum in einem Bereich der Farbe Rosa, der Farbe Blau bis zur Farbe Grün mit einer Wellenlänge von 400 bis 550 Nanometer liegt, und ein Farbwert der Oberflächenfarbe des Gehäuses (12) um das Befestigungsteil (14) herum, der durch ein Munsell-Farbsystem definiert ist, zwischen 1 und 4 liegt,
wobei das Befestigungsteil (14) so geformt ist, dass es aus der oberen Oberfläche (12b) des Gehäuses (12) hervorsteht,
wobei die horizontale Oberfläche (14c) des Befestigungsteils (14) zu einem hervorstehenden Teil (14e) des Befestigungsteils (14), der über eine Seite des Gehäuses (12) hinausragt, verläuft, und
das Befestigungsteil (14) eine Form hat, die in Richtung zum Inneren der Bluttestvorrichtung (11) schmäler wird.

2. Bluttestvorrichtung (11) nach Anspruch 1, die ferner einen Bedienknopf (16, 16a, 16b) aufweist, der mit dem elektrischen Schaltungsabschnitt (30) verbunden ist und der in der Oberfläche des Gehäuses vorgesehen ist,
wobei eine Oberflächenfarbe des Bedienknopfes einen Farbwert hat, der höher ist als der Farbwert der Oberflächenfarbe des Gehäuses um den Bedienknopf (16, 16a, 16b) herum, und die Farbe ist, die mit Augen mit beeinträchtigtem blauen Konus erkennbar ist,
wobei ein Farbton der Oberflächenfarbe des Bedienknopfes im Bereich der Farbe Orange bis zur Farbe Rot mit einer Wellenlänge von 600 bis 700 Nanometer liegt, und ein Farbwert der Oberflächenfarbe des Bedienknopfes, der durch das Munsell-Farbsystem definiert ist, zwischen 9,5 und 7 liegt.

3. Bluttestvorrichtung (11) nach Anspruch 1, wobei ein fluoreszierendes Material oder ein Phosphoreszierungsmittel auf das Befestigungsteil (14) aufgebracht ist.

4. Bluttestvorrichtung (11) nach Anspruch 2, wobei ein fluoreszierendes Material oder ein Phosphoreszierungsmittel auf den Bedienknopf (16, 16a, 16b) aufgebracht ist.

5. Bluttestvorrichtung nach Anspruch 1, wobei eine Leuchtdiode in dem Befestigungsteil (14) eingebettet ist.

6. Bluttestvorrichtung nach Anspruch 2, wobei eine Leuchtdiode in dem Bedienknopf (16, 16a, 16b) eingebettet ist.

7. Bluttestvorrichtung (11) nach Anspruch 1, das ausgebildet ist, einen Blutzuckerspiegel zu messen.

8. Bluttestvorrichtung (11) nach Anspruch 1, das zum Verwenden einer Kassette mit den Blutsensoren, die in der Bluttestvorrichtung (11) zu lagern sind, geeignet ist, wobei das Befestigungsteil (14) ausgebildet ist, die Blutsensoren einzeln vom Inneren der Bluttestvorrichtung (11) aus weiterzugeben.

## Revendications

1. Appareil d'analyse de sang (11) comprenant :
un boîtier (12); et
une section de circuit électrique (30) intégrée dans le boîtier (12) pour analyser un signal électrique provenant d'un capteur de sang ;
l'appareil d'analyse de sang (11) ayant une partie de fixation (14) pour fixer le capteur de sang, la partie de fixation (14) étant disposée dans une surface du boîtier (12),
dans lequel la partie de fixation (14) comprend une surface horizontale (14c), formée horizontalement par rapport à une surface supérieure (12b) du boîtier (12), et une surface de paroi dressée (14b) et une surface verticale (14d) qui sont formées verticalement par rapport à la surface supérieure (12b) du boîtier (12),
**caractérisé en ce que**
une couleur de surface d'une surface de la partie de fixation (14), où ladite surface est au moins l'une parmi la surface horizontale (14c), la surface de paroi dressée (14b) et la surface verticale (14d), a une valeur de couleur supérieure à une valeur de couleur d'une couleur de surface du boîtier (12) autour de la partie de fixation (14) et est une couleur qui peut être identifiée par les yeux avec des cônes bleus altérés,
une teinte de la couleur de surface de ladite surface de la partie de fixation (14) est dans une plage allant d'une couleur orange à une couleur rouge avec une longueur d'onde de 600 à 700 nanomètres, et une valeur de couleur de la couleur de surface de la partie de fixation (14) définie par le système de couleurs de Munsell est comprise entre 9,5 et 7,
une teinte de la couleur de surface du boîtier (12) autour de la partie de fixation (14) est dans une plage allant d'une couleur violette, d'une couleur bleue à une couleur verte avec une longueur d'onde de 400 à 550 nanomètres, et une valeur de couleur de la couleur de surface du boîtier (12) autour de la partie de fixation (14) définie par un système de couleurs de Munsell est comprise entre 1 et 4,
la partie de fixation (14) est formée pour faire saillie depuis la surface supérieure (12b) du boîtier (12),
la surface horizontale (14c) de la partie de fixation (14) s'étend jusqu'à une partie en saillie (14e) de la partie de fixation (14) faisant saillie au-delà d'un côté du boîtier (12), et
la partie de fixation (14) a une forme qui se rétrécit vers un intérieur de l'appareil d'analyse de sang (11).

2. Appareil d'analyse de sang (11) selon la revendication 1, comprenant en outre un bouton de fonctionnement (16, 16a, 16b) qui est connecté à la section de circuit électrique (30) et qui est disposé dans la surface du boîtier,
dans lequel une couleur de surface du bouton de fonctionnement a une valeur de couleur supérieure à la valeur de couleur de la couleur de surface du boîtier autour du bouton de fonctionnement (16, 16a, 16b) et est la couleur qui peut être identifiée par les yeux avec des cônes bleus altérés,
dans lequel la teinte de la couleur de surface du bouton de fonctionnement est dans une plage allant d'une couleur orange à une couleur rouge avec une longueur d'onde de 600 à 700 nanomètres, et une valeur de couleur de la couleur de surface du bouton de fonctionnement définie par le système de couleurs de Munsell est comprise entre 9,5 et 7.

3. Appareil d'analyse de sang (11) selon la revendication 1, dans lequel un matériau fluorescent ou un agent phosphorescent est appliqué à la partie de fixation (14).

4. Appareil d'analyse de sang (11) selon la revendication 2, dans lequel un matériau fluorescent ou un agent phosphorescent est appliqué au bouton de fonctionnement (16, 16a, 16b).

5. Appareil d'analyse de sang selon la revendication 1, dans lequel une diode électroluminescente est incorporée dans la partie de fixation (14).

6. Appareil d'analyse de sang selon la revendication 2, dans lequel une diode électroluminescente est incorporée dans le bouton de fonctionnement (16, 16a, 16b).

7. Appareil d'analyse de sang (11) selon la revendication 1, qui est adapté pour mesurer un niveau de sucre dans le sang.

8. Appareil d'analyse de sang (11) selon la revendication 1, destiné à utiliser une cartouche avec les capteurs de sang à stocker dans l'appareil d'analyse de sang (11), dans lequel la partie de fixation (14) est adaptée pour faire passer les capteurs de sang un par un depuis l'intérieur de l'appareil d'analyse de sang (11).
